(19) Europäisches Patentamt / European Patent Office / Office européen des brevets

(11) **EP 2 503 954 B1**

(12) **FASCICULE DE BREVET EUROPEEN**

(45) Date de publication et mention de la délivrance du brevet:
**09.10.2013 Bulletin 2013/41**

(21) Numéro de dépôt: **10803606.2**

(22) Date de dépôt: **24.11.2010**

(51) Int Cl.:
***A61B 8/08*** *(2006.01)*

(86) Numéro de dépôt international:
**PCT/FR2010/052505**

(87) Numéro de publication internationale:
**WO 2011/064498 (03.06.2011 Gazette 2011/22)**

(54) **PROCEDE ET DISPOSITIF DE CONTROLE ULTRASONORE DE LA TENUE MECANIQUE D'UNE PIECE INSEREE DANS UN CORPS, EN PARTICULIER D'UN IMPLANT DENTAIRE**

VERFAHREN UND VORRICHTUNG ZUM ULTRASCHALLTESTEN DER MECHANISCHEN FESTIGKEIT EINES IN EINEM KÖRPER EINGESETZTEN TEILS, IM BESONDEREN EINES ZAHNIMPLANTATS

METHOD AND DEVICE FOR ULTRASOUND TESTING OF THE MECHANICAL STRENGTH OF A PART INSERTED IN A BODY, IN PARTICULAR OF A DENTAL IMPLANT

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **24.11.2009 FR 0958325**

(43) Date de publication de la demande:
**03.10.2012 Bulletin 2012/40**

(73) Titulaires:
• **Université Paris Diderot - Paris 7**
**75205 Paris Cedex 13 (FR)**
• **CENTRE NATIONAL DE**
**LA RECHERCHE SCIENTIFIQUE (CNRS)**
**75016 Paris (FR)**

(72) Inventeurs:
• **HAIAT, Guillaume**
**94410 Saint Maurice (FR)**

• **MATTHIEU, Vincent**
**F-75013 Paris (FR)**
• **SOFFER, Jean Emmanuel**
**92320 Châtillon (FR)**
• **ANAGNOSTOU, Fani**
**F-75013 Paris (FR)**

(74) Mandataire: **Gendron, Vincent Christian et al**
**Fédit-Loriot**
**38, avenue Hoche**
**75008 Paris (FR)**

(56) Documents cités:
**WO-A2-01/37733      WO-A2-03/047451**
**WO-A2-2008/092125   US-A- 3 094 115**
**US-A1- 2004 077 949**

**Description**

La présente invention concerne le domaine du contrôle non destructif et se rapporte plus particulièrement à un procédé et un dispositif de contrôle de la tenue mécanique d'une pièce insérée au moins partiellement dans un corps, ladite pièce présentant une extrémité libre émergeant à la surface dudit corps à l'opposé d'une extrémité de ladite pièce enfouie dans ledit corps.

Il s'agit notamment, selon une application particulière de l'invention touchant au domaine de la technique chirurgicale implantaire, de contrôler la stabilité d'un implant, notamment d'un implant dentaire, ancré dans un os.

Concernant les implants dentaires, ce type d'implant se présente classiquement sous la forme d'une racine dentaire artificielle, généralement en titane ou en alliage contenant du titane, mise en place à l'intérieur de l'os de la mâchoire supérieure ou inférieure et sur laquelle vient se fixer un élément de prothèse dentaire.

Classiquement, après la pose de l'implant dentaire, une période de cicatrisation est nécessaire pour que les cellules osseuses viennent coloniser la surface implantée de l'implant et que celui-ci soit « ostéo-intégré », c'est-à-dire parfaitement intégré au niveau osseux, sans interposition de tissu fibreux au niveau de l'interface de contact entre l'os et l'implant. En effet, l'implant jouant le rôle d'intermédiaire entre l'élément de prothèse et l'os de la mâchoire et transmettant notamment les forces au support osseux, il doit être parfaitement intégré dans celui-ci.

Durant cette période, l'implant est donc susceptible d'être laissé au repos afin d'obtenir son intégration au niveau osseux, pour qu'il puisse être en mesure de supporter les charges qui s'exerceront sur lui. Le moment choisi par le chirurgien pour mettre en place l'élément de prothèse sur l'implant dentaire dépend de la durée de cette période de cicatrisation.

La mesure de la bonne ostéo-intégration de l'implant s'avère donc primordiale pour la réussite du traitement implantaire, en particulier afin de pouvoir déterminer précisément à quel moment un implant peut être chargé avec l'élément de prothèse.

Il est connu, par exemple du document US 5,392,779, un système de détermination de la stabilité d'un implant dentaire basé sur une mesure de la fréquence de résonance de l'implant. Ce système se compose d'un transducteur adapté à être connecté directement par vissage à l'implant et d'un appareil raccordé au transducteur, adapté à appliquer au transducteur un signal électrique dont la fréquence varie entre 5 et 15 kHz. La réponse du transducteur à la vibration due au signal correspond à la première fréquence de résonance perçue. Cette mesure en kHz est usuellement convertie en unités standardisées ISQ (pour « Implant Stability Quotient »), dont les valeurs, comprises entre 0 et 100, permettent d'évaluer la qualité de l'ancrage osseux implantaire. Une valeur proche de 0 correspond à une fréquence de résonance de faible amplitude et donc à un implant qui n'est pas fermement maintenu dans l'os. Inversement une valeur ISQ proche de 100 traduit une forte valeur de la fréquence de résonance et donc un implant solidement maintenu dans l'os.

Cependant, cette méthode pour déterminer la stabilité d'un implant dentaire n'est pas totalement satisfaisante. D'une part, elle permet uniquement d'évaluer la qualité de la tenue de l'implant au niveau global mais ne permet pas d'accéder à une analyse plus fine du phénomène d'ostéo-intégration, en particulier, concernant les propriétés biomécaniques de l'interface entre l'os et le titane de l'implant dentaire. Or, il existe un besoin d'une analyse quantitative plus localisée du phénomène d'intégration osseuse de l'implant pour la mise en place d'un protocole d'accompagnement efficace d'un traitement implantaire.

En outre, les différentes études faisant appel à l'analyse de la fréquence de résonance pour mesurer la stabilité implantaire montrent que la fréquence de résonance détectée dépend aussi de facteurs d'enfouissement de l'implant (importance de la surface de contact os-implant, hauteur non enfouie), de facteurs environnementaux (densité osseuse, micro et macro architecture de l'os autour de l'implant) ou encore de facteurs inhérents au vissage et au positionnement du transducteur, dont la corrélation avec la mesure de la fréquence de résonance est difficile à établir. Ces différents facteurs rendent donc délicate l'interprétation de la fréquence de résonance détectée pour évaluer valablement la stabilité d'un implant.

Dans ce contexte, la présente invention a pour but de proposer un procédé et un dispositif de contrôle de la stabilité d'un implant, notamment d'un implant dentaire, exempts de l'une au moins des limitations précédemment évoquées relatives aux techniques conventionnelles faisant appel à l'analyse de la fréquence de résonance de l'implant soumis à une sollicitation mécanique, en vue de l'établissement d'un diagnostic précis d'ostéo-intégration.

Plus généralement, un but de l'invention est de proposer un procédé et un dispositif de contrôle de la bonne tenue mécanique d'une pièce insérée dans un corps, qui permettent, en particulier, de fournir une estimation des propriétés mécaniques à l'interface de contact entre le corps et la pièce.

L'invention atteint son but en proposant un procédé de contrôle, par ailleurs conforme à la définition générique qu'en donne le préambule ci-dessus, essentiellement caractérisé en ce qu'il s'agit d'un procédé échographique ultrasonore comprenant des étapes dans lesquelles :

- on émet depuis l'extrémité libre de l'implant dentaire en direction de l'extrémité enfouie, à l'aide d'un transducteur ultrasonore, une onde ultrasonore de manière que l'onde ultrasonore se propage dans l'implant dentaire entre son extrémité libre et son extrémité enfouie, l'extrémité libre de l'implant dentaire étant utilisée comme site d'accueil pour positionner le transducteur ultrasonore ;

- on recueille, sous forme de signaux, des ondes ultrasonores réfléchies au niveau d'une interface de contact entre l'implant dentaire et l'os; et

- on traite lesdits signaux pour évaluer la quantité de tissus osseux en contact direct avec la surface externe de l'implant dentaire au niveau de l'interface de contact, de sorte à contrôler l'intégration osseuse de l'implant dentaire au niveau de l'os.

De préférence, la fréquence centrale de l'onde ultrasonore émise est choisie égale à au moins 8 MHz, avantageusement égale à au moins 10 MHz, et la bande passante, mesurée à -6 dB, est de l'ordre de 80% de la fréquence centrale.

Avantageusement, on utilise un transducteur ultrasonore piézoélectrique présentant une surface active de dimensions au moins égales à celles de la surface formée par ladite extrémité libre de ladite pièce et on positionne la surface active au contact de ladite extrémité libre et parallèlement à ladite surface formée par ladite extrémité libre de ladite pièce.

De préférence, on fournit une pièce d'adaptation se présentant sous la forme d'une bague apte à être montée solidaire du transducteur ultrasonore, ladite bague comprenant une surface intérieure circulaire destinée à venir en appui d'une part, contre une paroi latérale du transducteur ultrasonore s'étendant depuis ladite surface active du transducteur ultrasonore et, d'autre part, contre une paroi latérale de l'implant dentaire s'étendant depuis l'extrémité libre de l'implant dentaire, et on positionne ladite surface active du transducteur ultrasonore au contact de ladite extrémité libre de l'implant à l'aide de ladite pièce d'adaptation montée solidaire du transducteur ultrasonore, de manière à rendre le positionnement du transducteur ultrasonore par rapport à l'implant reproductible.

Selon un mode de réalisation, l'étape de traitement consiste à calculer une valeur moyenne temporelle, sur un intervalle de temps donné, à partir d'une pluralité de signaux recueillis consécutivement, de manière à générer un signal d'écho sur ledit intervalle de temps donné comportant une pluralité d'échos élémentaires résultant de la réflexion de l'onde ultrasonore émise sur ladite interface de contact et à traiter ledit signal d'écho pour déterminer un indicateur dont la valeur est représentative des propriétés mécaniques de ladite interface de contact.

Notamment, la détermination dudit indicateur peut consister à mesurer, pour chacun desdits échos élémentaires dudit signal d'écho, l'amplitude maximale correspondante dudit écho élémentaire et à calculer la moyenne des amplitudes maximales desdits échos élémentaires, ladite moyenne calculée définissant la qualité des propriétés mécaniques de l'interface de contact en termes de tenue mécanique.

Selon une variante, la détermination dudit indicateur consiste à mesurer l'amplitude maximale correspondante des échos élémentaires pairs dudit signal d'écho, et à calculer la moyenne des amplitudes maximales desdits échos élémentaires pairs, ladite moyenne calculée définissant la qualité des propriétés mécaniques de l'interface de contact en termes de tenue mécanique.

L'invention concerne également un dispositif de contrôle de la tenue mécanique d'un implant dentaire inséré au moins partiellement dans un os, l'implant dentaire présentant une extrémité libre affleurant à la surface de l'os et une extrémité enfouie dans l'os, opposée à ladite extrémité libre, caractérisé en ce qu'il comprend un transducteur ultrasonore présentant une surface active apte à être positionnée au contact de l'extrémité libre de l'implant dentaire, parallèlement à une surface définie par ladite extrémité libre de l'implant dentaire, de sorte à émettre une onde ultrasonore se propageant à l'intérieur de l'implant dentaire depuis l'extrémité libre vers l'extrémité enfouie, ledit transducteur ultrasonore étant apte à recueillir, sous forme de signaux, des ondes ultrasonores réfléchies au niveau d'une interface de contact entre l'implant dentaire et l'os, ledit dispositif comprenant en outre des moyens de mémorisation aptes à mémoriser les signaux recueillis représentatifs des ondes ultrasonores réfléchies et des moyens de traitement aptes à exécuter un traitement desdits signaux mémorisés pour évaluer la quantité de tissus osseux en contact direct avec la surface externe de l'implant dentaire au niveau de ladite interface de contact, de sorte à contrôler l'intégration osseuse de l'implant dentaire au niveau de l'os.

Selon un mode de réalisation particulier, le dispositif de contrôle peut comprendre une pièce d'adaptation se présentant sous la forme d'une bague apte à être montée solidaire du transducteur ultrasonore, ladite bague comprenant une surface intérieure circulaire destinée à venir en appui d'une part, contre une paroi latérale du transducteur ultrasonore s'étendant depuis ladite surface active du transducteur ultrasonore et, d'autre part, contre une paroi latérale de l'implant dentaire s'étendant depuis l'extrémité libre de l'implant dentaire. La pièce d'adaptation permet avantageusement d'optimiser le positionnement du transducteur par rapport à l'implant, notamment en ce favorisant le caractère reproductible de ce positionnement.

Selon une variante de réalisation, la surface intérieure circulaire de la bague présente une première surface intérieure circulaire apte à venir en appui contre ladite paroi latérale du transducteur ultrasonore, ladite première surface intérieure circulaire étant reliée par l'intermédiaire d'une surface de liaison sensiblement plane à une, deuxième surface intérieure circulaire de ladite bague, présentant un diamètre inférieur à celui formé par la première surface intérieure circulaire, et apte à venir en appui contre ladite paroi latérale de l'implant dentaire, la bague étant montée solidaire autour du transducteur ultrasonore de sorte à laisser un espace libre entre ladite surface active du transducteur et ladite surface de liaison sensiblement plane reliant lesdites première et deuxième surfaces intérieures circulaire de la bague. L'optimisation du positionnement du transducteur par rapport à l'implant peut ainsi être assurée, y compris dans les cas de figure où la surface active du transducteur présente un diamètre supérieur à celui de l'extrémité libre de l'implant.

De préférence, ladite pièce d'adaptation comprend des moyens de serrage pour maintenir en appui la surface intérieure circulaire de la bague contre la paroi latérale du transducteur ultrasonore.

De préférence, la fréquence centrale de l'onde ultrasonore émise par le transducteur ultrasonore est égale à au moins 8 MHz, avantageusement égale à au moins 10 MHz.

D'autres particularités et avantages de l'invention ressortiront à la lecture de la description faite ci-après d'un mode de réalisation particulier de l'invention, donné à titre indicatif mais non limitatif, en référence aux dessins annexés sur lesquels:

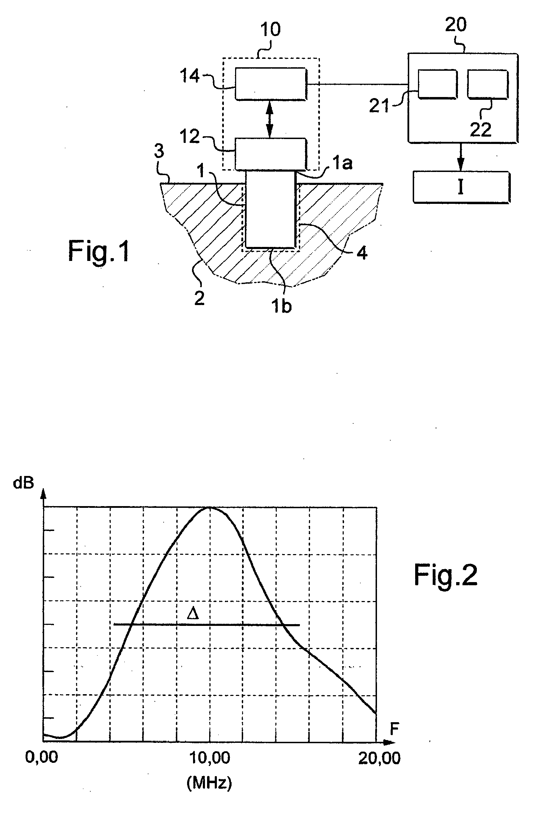

- la Figure 1 est une vue schématique d'ensemble d'un dispositif de contrôle selon l'invention ;
- la Figure 2 représente le spectre d'émission du signal correspondant à l'onde ultrasonore utilisée ;

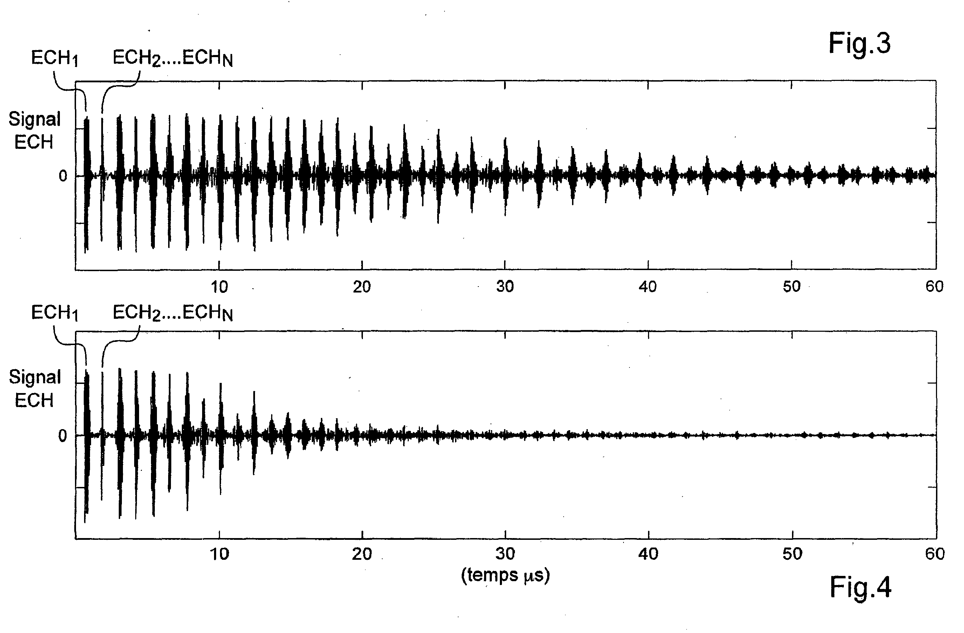

- la Figure 3 est un exemple d'échogramme obtenu lors du contrôle de la tenue mécanique de l'implant de la figure 1 dans un premier cas de figure où la zone d'interface de contact entre l'os et l'implant génère une forte discontinuité entre les deux milieux, ce qui est par exemple le cas lors de la présence de tissu fibreux à l'interface;

- la Figure 4 est un exemple d'échogramme obtenu lors du contrôle de la tenue mécanique de l'implant de la figure 1 dans un second cas de figure où la zone d'interface de contact entre l'os et l'implant génère une plus faible discontinuité entre les deux milieux, ce qui est par exemple le cas lors de l'absence de tissu fibreux à l'interface;

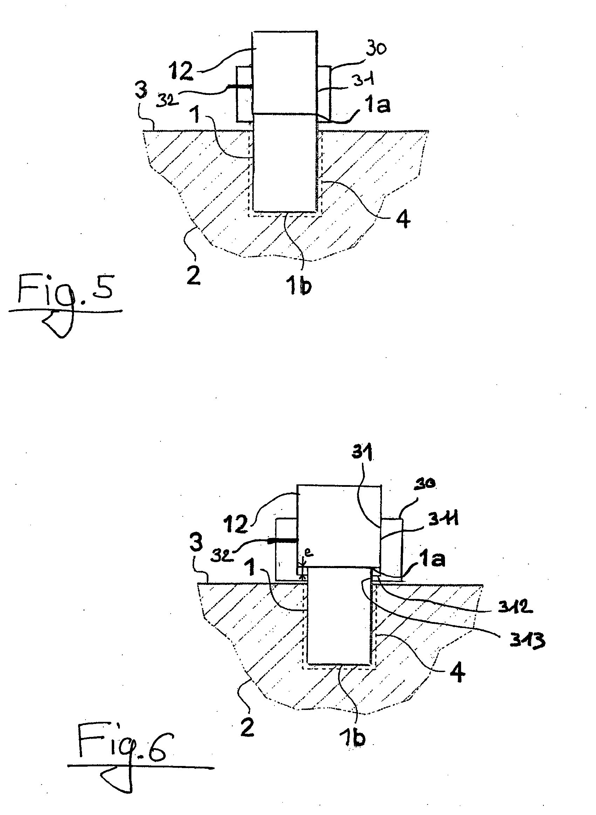

- Les Figures 5 et 6 illustrent une vue de détail de la liaison entre le transducteur ultrasonore et l'extrémité libre de l'implant dentaire, selon deux variantes d'un mode de réalisation particulier où une pièce d'adaptation équipe le transducteur ultrasonore, visant à garantir un positionnement axial précis et reproductible du transducteur ultrasonore relativement à l'extrémité libre de l'implant.

Le phénomène de propagation des ultrasons au sein de l'implant est directement lié aux conditions d'interaction des ultrasons à l'interface de contact entre l'os et l'implant. Or, lorsque des ondes ultrasonores rencontrent une interface délimitant deux milieux ayant des propriétés mécaniques différentes, il y a réflexion (avec éventuellement conversion de mode) et les ondes réfléchies donnent lieu à un écho, selon le principe de l'échographie ultrasonore.

On propose donc d'utiliser ce principe de l'échographie ultrasonore pour estimer les conditions aux limites au niveau de cette zone d'interface de contact entre l'os et l'implant, en particulier au niveau des parois latérales de l'implant et de définir une méthode permettant, à partir de cette estimation, de caractériser les propriétés biomécaniques de cette zone d'interface de contact pour fournir une indication sur la qualité de la stabilité de l'implant notamment.

Pour ce faire, la figure 1 décrit schématiquement une vue d'ensemble d'un dispositif pour la mise en oeuvre du procédé de contrôle selon l'invention. Selon l'exemple de la figure 1, la pièce contrôlée est un implant 1, tel qu'un implant dentaire en titane, inséré dans un os 2. L'implant dentaire 1 est modélisé sous la forme d'un cylindre et comprend par exemple des dimensions de 4 mm de diamètre et de 7 mm de longueur. Une fois ancré dans l'os 2, l'implant dentaire 1 présente une extrémité libre la définissant une surface circulaire (présentant par exemple un diamètre égal à 5 mm) émergeant à la surface 3 de l'os 2, à l'opposé d'une extrémité 1 b de l'implant enfouie dans l'os 2, de sorte que cette extrémité libre de l'implant dentaire 1 est susceptible d'être accessible in vivo.

Le dispositif de contrôle comprend principalement un capteur ultrasonore 10 et une unité de calcul 20. Le capteur ultrasonore, de type piézo-électrique, comporte un transducteur ultrasonore 12 pour émettre une onde ultrasonore en régime impulsionnel et recevoir les échos résultant de la réflexion de l'onde ultrasonore se propageant dans l'implant et un circuit de commande 14 pour commander le transducteur ultrasonore.

Le transducteur ultrasonore est prévu pour être utilisé en mode échographique et est placé de sorte que sa surface active, de dimensions au moins égales à celles de la surface formée par l'extrémité libre de l'implant, soit positionnée directement au contact de l'extrémité libre la de l'implant et sensiblement parallèlement à la surface de l'implant définie par son extrémité libre. L'extrémité libre de l'implant, lorsque celle-ci est facilement accessible, est ainsi avantageusement utilisée comme site d'accueil pour positionner le transducteur ultrasonore. Le transducteur est de préférence couplé à l'extrémité libre de l'implant par l'intermédiaire d'un gel ultrasonore fournissant des conditions adaptées de propagation des ultrasons à l'interface de contact entre la surface active du transducteur et l'extrémité libre de l'implant.

Selon un mode de réalisation particulier décrit en référence aux figures 5 et 6 illustrant une vue de détail de la liaison entre le transducteur ultrasonore et l'implant dentaire telle qu'illustrée schématiquement à la figure 1, une pièce d'adaptation 30 est prévue pour être montée solidaire du transducteur ultrasonore 12, autour de l'extrémité du transducteur située du côté de la surface active destinée à venir en contact avec l'extrémité libre 1a de l'implant dentaire 1. Cette pièce d'adaptation 30 présente une double fonction. Elle permet de monter le transducteur ultrasonore 12 au contact de l'extrémité libre 1a de l'implant dentaire 1 d'une manière d'une part, qui minimise les mouvements du transducteur ultrasonore par rapport à l'implant et, d'autre part, qui soit reproductible, en termes de positionnement du transducteur ultrasonore et de l'implant dentaire l'un relativement à l'autre et, en particulier, en termes d'angle de positionnement de la surface active du transducteur ultrasonore relativement à l'extrémité libre de l'implant dentaire.

La pièce d'adaptation 30 se présente sous la forme d'une bague apte à entourer le transducteur ultrasonore 12. La bague comprend une surface intérieure circulaire 31 destinée à venir en appui d'une part, contre la paroi latérale du transducteur ultrasonore 12, s'étendant depuis la surface active du transducteur ultrasonore et, d'autre part, contre la paroi latérale de l'implant dentaire 1, s'étendant depuis son extrémité libre 1 a.

La bague présente avantageusement des moyens de serrage 32 pour maintenir en appui la surface intérieure circulaire 31 contre la paroi latérale du transducteur ultrasonore. Les moyens de serrage 32 permettent ainsi de maintenir fermement la bague au niveau du transducteur ultrasonore. Pour ce faire, la bague est par exemple tronquée pour former deux extrémités libres en regard et ces deux extrémités libres sont entraînées à force l'une vers l'autre avec des moyens élastiques par l'intermédiaire d'une vis de serrage.

Comme illustré à la figure 5, la surface active du transducteur ultrasonore 12 présente un diamètre identique à celui de l'extrémité libre 1a de l'implant 1. Dans ce cas de figure, la surface intérieure circulaire de la bague présente un même diamètre sur toute la hauteur de la bague, à savoir un diamètre sensiblement égal à celui défini à la fois par le transducteur ultrasonore et par l'extrémité libre de l'implant.

Toutefois, lorsque la surface active du transducteur ultrasonore 12 présente un diamètre supérieur à celui de l'extrémité libre 1a de l'implant 1, comme illustré à la figure 1 et à la figure 6, la bague formant la pièce d'adaptation 30 nécessite d'être configurée différemment. En particulier, la surface intérieure circulaire 31 de la bague présente une première surface intérieure circulaire 311 destinée à être engagée autour de la paroi latérale du transducteur ultrasonore, reliée par l'intermédiaire d'une surface de liaison 312 sensiblement plane à une deuxième surface intérieure circulaire 313, de diamètre inférieur à celui formé par là première surface intérieure circulaire 311, et destinée à être engagée autour de la paroi latérale de l'implant au niveau de son extrémité libre. En outre, la bague est montée autour du transducteur ultrasonore de sorte à laisser un espace libre e entre la surface active du transducteur et la surface de liaison 312 sensiblement plane reliant les première et deuxième surfaces intérieures circulaire de la bague. Cet espace libre, rempli d'air, permet avantageusement d'éviter toutes réflexions parasites de l'onde ultrasonore sur la pièce d'adaptation lors de la mise en oeuvre de la méthode de contrôle de la tenue mécanique de l'implant dentaire, qui sera expliquée plus en détail ci- après.

Dans ces conditions, les ondes ultrasonores émises peuvent se propager à l'intérieur de l'implant, perpendiculairement à la surface active du transducteur, depuis l'extrémité libre 1a de l'implant vers son extrémité enfouie 1b, tout en favorisant le phénomène d'interactions des ondes ultrasonores se propageant à l'intérieur de l'implant avec une interface de contact 4 située le long de la frontière entre la surface externe de l'implant et l'os entourant celui-ci. Ce phénomène d'interaction est en effet essentiel pour la mise en oeuvre du procédé selon l'invention, comme il apparaîtra par la suite.

Le circuit de commande 14 comprend un générateur d'impulsions électriques utilisé pour exciter l'élément piézo-électrique du transducteur, lequel transforme les impulsions électriques reçues en ondes ultrasonores correspondantes, qui se propagent dans l'implant via la surface supérieure de l'implant. Le générateur d'impulsions électriques utilisé pour exciter le transducteur utilise des formes d'ondes du type impulsion brève ou signal carré à fronts de montée et de descente rapides.

Le circuit de commande 14 comprend également un circuit de réception recevant le signal électrique délivré par le transducteur ultrasonore correspondant aux ondes ultrasonores réfléchies au niveau de l'interface de contact 4, reçues par le transducteur ultrasonore.

L'unité de calcul 20, reliée au capteur 10, par exemple par l'intermédiaire d'une ligne de transmission de type coaxiale, comprend principalement des moyens de mémorisation numériques 21 aptes à mémoriser les signaux électriques représentatifs des ondes ultrasonores réfléchies, recueillis par le circuit de réception du circuit de commande, et des moyens de traitement 22 aptes à exécuter un traitement de ces signaux mémorisés pour mesurer les propriétés mécaniques de l'interface de contact entre l'implant et l'os, comme il sera vu en détail par la suite. Cette unité de calcul est avantageusement un ordinateur individuel. Divers équipements peuvent être ajoutés, tels qu'un moyen d'affichage et un moyen d'impression.

Compte tenu de l'application visée, la fréquence centrale de l'onde ultrasonore émise est choisie égale à au moins 8 MHz et la bande passante, mesurée à -6 dB, est de l'ordre de 80% de la fréquence centrale. De préférence, la limite inférieure de la bande passante est supérieure ou égale à 5 MHz. La figure 3 montre un spectre d'émission préférentiel du signal correspondant à l'onde ultrasonore utilisée avec une fréquence centrale égale à 10 MHz et une bande passante Δ comprise entre 6 et 14 MHz. De cette manière, on s'assure de pouvoir discriminer d'une façon suffisante les signaux réfléchis par l'interface de contact 4 en vue de leur traitement. La largeur d'impulsion est de préférence choisie inférieure ou égale à 1 μs.

Le traitement de signal comprend plusieurs étapes. Dans un premier temps, un traitement particulier est mis en oeuvre permettant de diminuer le bruit par moyennage temporel. Ce traitement consiste à calculer une valeur moyenne temporelle, sur un intervalle de temps donné, à partir d'une pluralité de signaux recueillis consécutivement, de manière à générer un signal d'écho sur cet intervalle de temps donné. Selon un exemple de réalisation, les signaux recueillis peuvent être enregistrés toutes les 10 ms, sur une durée égale à 60 µs et 500 signaux consécutifs ainsi enregistrés sont automatiquement moyennés. Ce nombre de signaux consécutifs correspond à un compromis entre une réduction significative du bruit et un temps de calcul raisonnable.

Les figures 3 et 4 illustrent deux exemples d'échogrammes sur lesquels apparait un signal d'écho ainsi obtenu, respectivement dans un premier cas où l'implant, de 4 mm de diamètre selon l'exemple, est inséré dans un trou de 4,8 mm de diamètre creusé dans l'os et dans un second cas où l'implant est inséré dans un trou de 4 mm de diamètre creusé dans l'os. Le signal d'écho ECH comporte une pluralité d'échos élémentaires successifs, ECH1 à ECHN, qui, comme il a été analysé, résultent des phénomènes d'interaction des ondes ultrasonores se propageant dans l'implant avec l'interface de contact 4 située le long de la frontière entre l'implant et l'os, en particulier autour de l'implant au niveau de ses parois latérales.

En effet, lors de la propagation de l'onde ultrasonore émise par le transducteur à l'intérieur de l'implant, il apparaît, en plus du mode normal de propagation longitudinale, un mode de propagation transversale généré par des effets de conversion de mode au niveau de l'interface de contact. Ces ondes transversales subissent elles mêmes des effets de conversion de mode transversal-longitudinal lors de leur propagation dans l'implant et interaction avec l'interface de contact. Ces effets de conversion de mode (de mode longitudinal à transversal et de mode transversal à longitudinal) au sein de l'implant expliquent la pluralité d'échos élémentaires successivement obtenus dans le signal d'écho.

L'étape suivante du traitement du signal vise alors à fournir une interprétation du signal d'écho ECH, à même de caractériser efficacement les propriétés mécaniques de l'interface de contact 4, et ainsi fournir un diagnostic quant à la stabilité de l'implant.

Il apparaît de la comparaison entre les échogrammes des figures 3 et 4 que l'amplitude des échos élémentaires $ECH_1$ à $ECH_N$ diminue moins rapidement dans le premier cas (Figure 3), où l'implant est inséré dans un trou de diamètre plus grand que le diamètre de l'implant. Autrement dit, l'amplitude des échos élémentaires du signal d'écho diminue moins rapidement quand la surface externe latérale de l'implant est entourée d'eau (ou de tissus fibreux) que quand elle est entourée directement d'os.

Les résultats obtenus suggèrent donc que l'atténuation des échos élémentaires du signal d'écho généré est susceptible d'être utilisée comme un indicateur significatif des propriétés mécaniques de l'interface de contact 4 le long des parois latérales de l'implant, en ce que cette atténuation est étroitement liée à la quantité d'os directement en contact avec la surface externe latérale de l'implant au niveau de l'interface de contact. En effet, plus il y a d'os en contact direct avec la surface externe latérale de l'implant au niveau de cette interface de contact, moins la discontinuité moyenne des propriétés mécaniques entre le titane de l'implant et l'os est importante et donc, moins les ondes ultrasonores sont réfléchies par l'interface, ce qui se traduit par une diminution des échos élémentaires dans le signal d'écho généré (Figure 4).

Aussi, pour déterminer un indicateur I dont la valeur est représentative des propriétés mécaniques de l'interface de contact 4, en particulier le long de la surface externe latérale de l'implant on mesure, pour chacun des échos élémentaires $ECH_1$ à $ECH_N$ identifiés dans le signal d'écho ECH, l'amplitude maximale correspondante, respectivement $A_1$ à $A_N$, puis on calcule la moyenne des amplitudes maximales des échos élémentaires qui ont été identifiés. L'indicateur I est donc donné par :

$$ I = \sum_{p=1}^{N} A_p $$

La valeur de cet indicateur permet ainsi de fournir une indication précise sur la qualité des propriétés mécaniques de l'interface de contact le long de la surface externe latérale de l'implant insérée dans l'os, en termes de tenue mécanique.

En effet, une valeur élevée de l'indicateur I traduit une présence importante d'échos élémentaires d'amplitude significative, ce qui indique une faible quantité de tissus osseux en contact direct avec la surface externe latérale de l'implant au niveau de l'interface de contact et donc des propriétés mécaniques de cette interface de contact faibles.

A l'inverse, une valeur plus faible de l'indicateur I traduit une moins grande quantité échos élémentaires d'amplitude significative, ce qui traduit une plus grande quantité de tissus osseux en contact direct avec la surface externe latérale de l'implant au niveau de l'interface de contact et donc des propriétés mécaniques de cette interface de contact plus élevée, le long de la surface externe latérale de l'implant.

En variante, plutôt que de considérer l'amplitude de chacun des échos élémentaires identifiés dans le signal d'écho généré pour calculer l'indicateur 1 comme expliqué ci-dessus, on prend uniquement en compte l'amplitude d'un écho élémentaire sur deux, à savoir l'amplitude des seuls échos pairs.

Ainsi, selon cette variante, l'indicateur I est calculé de la manière suivante :

$$ I = \sum_{p=1}^{N/2} A_{2.p} $$

Selon cette variante de calcul de l'indicateur I, il a été démontré expérimentalement et physiquement par la demanderesse qu'on obtenait des résultats encore mieux corrélés avec la quantité d'os en contact direct avec la surface externe latérale de l'implant.

Aussi, l'utilisation et l'analyse échographique de la propagation des ondes ultrasonores dans l'implant ainsi que de leurs interactions avec l'interface de contact entre l'implant et l'os, selon les principes de l'invention exposés ci-dessus, permettent de contrôler la tenue mécanique de l'implant par une méthode de détermination précise des propriétés mécaniques de l'interface de contact entre l'os et l'implant, révélant la quantité de tissus osseux en contact direct avec la surface externe latérale de l'implant.

Le procédé de l'invention offre donc un outil de contrôle précis, fiable et simple de mise en oeuvre de l'évolution du processus d'ostéo-intégration.

Bien que la description qui précède ait été faite en référence à un implant dentaire, la chirurgie orthopédique est également susceptible de constituer un champ d'application de l'invention. En effet, des vis orthopédiques sont régulièrement posées et leur tenue mécanique dans l'os peut également être estimée par le procédé de l'invention, conformément aux principes exposés ci-dessus.

En outre, d'autres domaines d'applications peuvent également être envisagés dans le domaine du contrôle non destructif par ultrasons. Il peut par exemple s'agir d'estimer la tenue mécanique de vis, clous ou rivets insérés dans un milieu complexe soumis à des modifications, par exemple à cause des conditions extérieures. Les besoins se situent notamment pour les applications où de fortes exigences de sécurité sont rencontrées comme dans les industries aéronautique, ferroviaire ou nucléaire.

**Revendications**

1. Procédé de contrôle de la tenue mécanique d'un implant dentaire (1) inséré au moins partiellement dans un os (2), l'implant dentaire se présentant sous la forme d'une pièce sensiblement cylindrique réalisée en titane ou en un alliage contenant du titane et présentant une extrémité libre (1 a) émergeant à la surface de l'os à l'opposé d'une extrémité (1b) de l'implant dentaire enfouie dans l'os, **caractérisé en ce qu'**il s'agit d'un procédé échographique ultrasonore comprenant des étapes dans lesquelles :

   - on positionne une surface active d'un transducteur ultrasonore (12) au contact de ladite extrémité libre (1a) et parallèlement à la surface formée par ladite extrémité libre de l'implant dentaire ;
   - on émet, à l'aide dudit transducteur ultrasonore (12), des ondes ultrasonores se propageant dans l'implant dentaire (1) selon une direction de propagation perpendiculaire à ladite surface active dudit transducteur ultrasonore, depuis ladite extrémité libre de l'implant dentaire vers son extrémité enfouie,;
   - on recueille à l'aide dudit transducteur ultrasonore, sous forme de signaux, des ondes ultrasonores réfléchies résultant de l'interaction desdites ondes ultrasonores se propageant dans l'implant dentaire avec une interface de contact (4) entre l'implant dentaire et l'os, située le long d'une frontière entre la surface externe de l'implant dentaire et l'os au niveau des parois latérales de l'implant dentaire; et
   - on traite lesdits signaux pour évaluer la quantité de tissus osseux en contact direct avec la surface externe de l'implant dentaire au niveau de ladite interface de contact, de sorte à contrôler l'intégration osseuse de l'implant dentaire au niveau de l'os.

2. Procédé selon la revendication 1, **caractérisé en ce que** la fréquence centrale de l'onde ultrasonore émise est choisie égale à au moins 8 MHz, avantageusement égale à au moins 10 MHz, et **en ce que** la bande passante, mesurée à -6 dB, est de l'ordre de 80% de la fréquence centrale.

3. Procédé selon la revendication 1 ou 2, **caractérisé en ce qu'**on utilise un transducteur ultrasonore (12) piézoélectrique présentant une surface active de dimensions au moins égales à celles de la surface formée par ladite extrémité libre (1a) de l'implant dentaire.

4. Procédé selon la revendication 3, **caractérisé en ce qu'**on fournit une pièce d'adaptation (30) apte à être montée solidaire du transducteur ultrasonore (12), se présentant sous la forme d'une bague comprenant une surface intérieure circulaire (31) destinée à venir en appui d'une part, contre une paroi latérale du transducteur ultrasonore (12) s'étendant depuis ladite surface active et, d'autre part, contre une paroi latérale de l'implant dentaire (1) s'étendant depuis l'extrémité libre (1a) de l'implant dentaire (1), et **en ce qu'**on positionne ladite surface active du transducteur ultrasonore au contact de ladite extrémité libre de l'implant dentaire à l'aide de ladite pièce d'adaptation (30) montée solidaire du transducteur ultrasonore (12), de manière à rendre le positionnement du transducteur ultrasonore par rapport à l'implant reproductible.

5. Procédé selon l'une quelconque des revendications précédentes, **caractérisé en ce que** l'étape de traitement consiste à calculer une valeur moyenne temporelle, sur un intervalle de temps donné, à partir d'une pluralité de signaux recueillis consécutivement, de manière à générer un signal d'écho (ECH) sur ledit intervalle de temps donné comportant une pluralité d'échos élémentaires (ECH$_1$,..., ECH$_N$) résultant de l'interaction desdites ondes ultrasonores se propageant dans l'implant avec ladite interface de contact (4) et à traiter ledit signal d'écho pour déterminer un indicateur (I) dont la valeur est représentative des propriétés mécaniques de ladite interface de contact (4).

6. Procédé selon la revendication 5, **caractérisé en ce que** la détermination dudit indicateur (I) consiste à mesurer, pour chacun desdits échos élémentaires (ECH$_1$,..., ECH$_N$) dudit signal d'écho (ECH), l'amplitude maximale correspondante dudit écho élémentaire et à calculer la moyenne des amplitudes maximales desdits échos élémentaires, ladite moyenne calculée définissant la qualité des propriétés mécaniques de l'interface de contact en termes de tenue mécanique.

7. Procédé selon la revendication 5, **caractérisé en ce que** la détermination dudit indicateur (I) consiste à mesurer l'amplitude maximale correspondante des échos élémentaires pairs dudit signal d'écho (ECH), et à calculer la moyenne des amplitudes maximales desdits échos élémentaires pairs, ladite moyenne calculée définissant la qualité des propriétés mécaniques de l'interface de contact en termes de tenue mécanique.

8. Dispositif de contrôle de la tenue mécanique d'un implant dentaire (1) inséré au moins partiellement dans un os (2), l'implant dentaire se présentant sous la forme d'une pièce sensiblement cylindrique réalisée en titane ou en un alliage contenant du titane et présentant une extrémité libre (1a) émergeant à la surface de l'os et une extrémité enfouie (1b) dans l'os, opposée à ladite extrémité libre, **caractérisé en ce qu'**il comprend un transducteur ultrasonore (12) présentant une surface active apte à être positionnée au contact de l'extrémité libre de l'implant dentaire, parallèlement à une surface définie par ladite extrémité libre de l'implant dentaire, de sorte à émettre des ondes ultrasonores se propageant à l'intérieur de l'implant dentaire selon une direction de propagation perpendiculaire à ladite surface active du transducteur ultrasonore, depuis ladite extrémité libre vers ladite extrémité enfouie dudit implant dentaire, ledit transducteur ultrasonore étant apte à recueillir, sous forme de signaux, des ondes ultrasonores réfléchies résultant de l'interaction desdites ondes ultrasonores se propageant dans l'implant avec une interface de contact (4) entre l'implant dentaire et l'os, située le long d'une frontière entre la surface externe de l'implant dentaire et l'os au niveau des parois latérales de l'implant dentaire, ledit dispositif comprenant en outre des moyens de mémorisation (21) aptes à mémoriser lesdits signaux recueillis représentatifs desdites ondes ultrasonores réfléchies et des moyens de traitement (22) aptes à exécuter un traitement desdits signaux mémorisés pour évaluer la quantité de tissus osseux en contact direct avec la surface externe de l'implant dentaire au niveau de ladite interface de contact, de sorte à contrôler l'intégration osseuse de l'implant dentaire au niveau de l'os.

9. Dispositif selon la revendication 8, **caractérisé en ce qu'**il comprend une pièce d'adaptation (30) se présentant sous la forme d'une bague apte être montée solidaire du transducteur ultrasonore (12), ladite bague comprenant une surface intérieure circulaire (31) destinée à venir en appui d'une part, contre une paroi latérale du transducteur ultrasonore (12) s'étendant depuis ladite surface active du transducteur ultrasonore et, d'autre part, contre une paroi latérale de l'implant dentaire (1) s'étendant depuis l'extrémité libre (1a) de l'implant dentaire.

10. Dispositif selon la revendication 9, **caractérisé en ce que** la surface intérieure circulaire (31) de la bague présente une première surface intérieure circulaire (311) apte à venir en appui contre ladite paroi latérale du transducteur ultrasonore, ladite première surface intérieure circulaire étant reliée par l'intermédiaire d'une surface de liaison (312) sensiblement plane à une deuxième surface intérieure circulaire (313) de ladite bague, présentant un diamètre inférieur à celui formé par la première surface intérieure circulaire (311), et apte à venir en appui contre ladite paroi latérale de l'implant dentaire (1), la bague étant montée solidaire autour du transducteur ultrasonore de sorte à laisser un espace libre (e) entre ladite surface active du transducteur (12) et ladite surface de liaison (312) sensiblement plane reliant lesdites première et deuxième surfaces intérieures circulaires de la bague.

11. Dispositif selon la revendication 9 ou 10, **caractérisé en ce que** ladite pièce d'adaptation (30) comprend des moyens de serrage (32) apte à maintenir en appui la surface intérieure circulaire (31) de la bague contre ladite paroi latérale du transducteur ultrasonore (12).

12. Dispositif selon l'une quelconque des revendications 8 à 11, **caractérisé en ce que** la fréquence centrale de l'onde ultrasonore émise par le transducteur ultrasonore est égale à au moins 8 MHz, avantageusement égale à au moins 10 MHz.

**Patentansprüche**

1. Verfahren zum Kontrollieren der mechanischen Festigkeit eines Zahnimplantats (1), das wenigstens teilweise in einen Knochen (2) eingesetzt ist, wobei das Zahnimplantat die Form eines im Wesentlichen zylindrischen Teils aufweist, das aus Titan oder aus einer Titan enthaltenden Legierung hergestellt ist und ein von der Oberfläche des Knochens vorstehendes freies Ende (1a) gegenüber einem im Knochen verborgenen Ende (1b) des Zahnimplantats aufweist, **dadurch gekennzeichnet, dass** es sich um ein Ultraschall-Echographieverfahren handelt, das die Schritte umfasst, in denen:

- eine aktive Oberfläche eines Ultraschallwandlers (12) in Kontakt mit dem freien Ende (1a) und parallel zu der durch das freie Ende des Zahnimplantats gebildeten Oberfläche positioniert wird;

- mit Hilfe des Ultraschallwandlers (12) Ultraschallwellen, die sich im Zahnimplantat (1) in einer zu der aktiven Oberfläche des Ultraschallwandlers senkrechten Ausbreitungsrichtung ausbreiten, von dem freien Ende des Zahnimplantats zu seinem verborgenen Ende ausgesendet werden;

- mit Hilfe des Ultraschallwandlers reflektierte Ultraschallwellen, die sich aus der Wechselwirkung der im Zahnimplantat sich ausbreitenden Ultraschallwellen mit einer Kontaktgrenzfläche (4) zwischen dem Zahnimplantat und dem Knochen, die sich entlang einer Grenze zwischen der äußeren Oberfläche des Zahnimplantats und dem Knochen auf Höhe der Seitenwände des Zahnimplantats befindet, ergeben, in Form von Signalen erfasst werden; und

- die Signale verarbeitet werden, um die Menge von Knochengewebe, die mit der äußeren Oberfläche des Zahnimplantats auf Höhe der Kontaktgrenzfläche in direktem Kontakt ist, einzuschätzen, derart, dass die Integration des Zahnimplantats in den Knochen auf Höhe des Knochens kontrolliert wird.

2. Verfahren nach Anspruch 1, **dadurch gekennzeichnet, dass** die Mittenfrequenz der ausgesendeten Ultraschallwelle wenigstens gleich 8 MHz, vorteilhaft wenigstens gleich 10 MHz, gewählt wird und dass das Durchlassband, das bei -6 dB gemessen wird, in der Größenordnung von 80 % der Mittenfrequenz liegt.

3. Verfahren nach Anspruch 1 oder 2, **dadurch gekennzeichnet, dass** ein piezoelektrischer Ultraschallwandler (12) verwendet wird, der eine aktive Oberfläche aufweist, die Abmessungen besitzt, die wenigstens gleich jenen der durch das freie Ende (1a) des Zahnimplantats gebildeten Oberfläche sind.

4. Verfahren nach Anspruch 3, **dadurch gekenn-**

**zeichnet, dass** ein Anpassungsteil (30) vorgesehen wird, das mit dem Ultraschallwandler (12) fest montiert werden kann und das die Form eines Rings aufweist, der eine kreisförmige innere Oberfläche (31) aufweist, die sich einerseits an einer Seitenwand des Ultraschallwandlers (12) abstützen soll, die sich von der aktiven Oberfläche erstreckt, und andererseits an einer Seitenwand des Zahnimplantats (1) abstützen soll, die sich von dem freien Ende (1a) des Zahnimplantats (1) erstreckt, und dass die aktive Oberfläche des Ultraschallwandlers in Kontakt mit dem freien Ende des Zahnimplantats mit Hilfe des Anpassungsteils (30), das fest an dem Ultraschallwandler (12) montiert ist, positioniert wird, derart, dass die Positionierung des Ultraschallwandlers in Bezug auf das Implantat reproduzierbar gemacht wird.

5. Verfahren nach einem der vorhergehenden Ansprüche, **dadurch gekennzeichnet, dass** der Verarbeitungsschritt darin besteht, einen zeitlichen Mittelwert in einem gegebenen Zeitintervall anhand mehrerer nacheinander erfasster Signale zu berechnen, derart, dass ein Echosignal (ECH) in dem gegebenen Zeitintervall erzeugt wird, das mehrere Elementarechos ($ECH_1$, ..., $ECH_N$) enthält, die sich aus der Wechselwirkung der in dem Implantat sich ausbreitenden Ultraschallwellen mit der Kontaktgrenzfläche (4) ergeben, und das Echosignal zu verarbeiten, um einen Indikator (I) zu bestimmen, dessen Wert mechanische Eigenschaften der Kontaktgrenzfläche (4) repräsentiert.

6. Verfahren nach Anspruch 5, **dadurch gekennzeichnet, dass** die Bestimmung des Indikators (I) darin besteht, für jedes der Elementarechos ($ECH_1$, ..., $ECH_N$) des Echosignals (ECH) die maximale Amplitude zu messen, die dem Elementarecho entspricht, und den Mittelwert maximaler Amplituden der Elementarechos zu berechnen, wobei der berechnete Mittelwert die Qualität der mechanischen Eigenschaften der Kontaktgrenzfläche ausgedrückt durch die mechanische Festigkeit definiert.

7. Verfahren nach Anspruch 5, **dadurch gekennzeichnet, dass** die Bestimmung des Indikators (I) darin besteht, die maximale Amplitude zu messen, die Elementarechopaaren des Echosignals (ECH) entspricht, und den Mittelwert der maximalen Amplituden der Elementarechopaare zu berechnen, wobei der berechnete Mittelwert die Qualität der mechanischen Eigenschaften der Kontaktgrenzfläche ausgedrückt durch die mechanische Festigkeit definiert.

8. Vorrichtung zum Kontrollieren der mechanischen Festigkeit eines Zahnimplantats (1), das wenigstens teilweise in einen Knochen (2) eingesetzt ist, wobei das Zahnimplantat die Form eines im Wesentlichen zylindrischen Teils aufweist, das aus Titan oder aus einer Titan enthaltenden Legierung hergestellt ist und ein von der Oberfläche des Knochens hervortretendes freies Ende (1a) und ein im Knochen verborgenes Ende (1b) gegenüber dem freien Ende aufweist, **dadurch gekennzeichnet, dass** sie einen Ultraschallwandler (12) umfasst, der eine aktive Oberfläche aufweist, die in Kontakt mit dem freien Ende des Zahnimplantats parallel zu einer durch das freie Ende des Zahnimplantats definierten Oberfläche positioniert werden kann, derart, dass er Ultraschallwellen, die sich in dem Zahnimplantat in einer zu der aktiven Oberfläche des Ultraschallwandlers senkrechten Ausbreitungsrichtung ausbreiten, von dem freien Ende zu dem verborgenen Ende des Zahnimplantats aussendet, wobei der Ultraschallwandler dafür ausgelegt ist, reflektierte Ultraschallwellen, die sich aus der Wechselwirkung der Ultraschallwellen, die sich im Implantat ausbreiten, mit einer Kontaktgrenzfläche (4) zwischen dem Zahnimplantat und dem Knochen, die sich entlang einer Grenze zwischen der äußeren Oberfläche des Zahnimplantats und dem Knochen auf Höhe der Seitenwände des Zahnimplantats befindet, ergeben, in Form von Signalen zu erfassen, wobei die Vorrichtung außerdem Speichermittel (21) umfasst, die dafür ausgelegt sind, erfasste Signale, die die reflektierten Ultraschallwellen repräsentieren, zu speichern, und Verarbeitungsmittel (22) umfasst, die dafür ausgelegt sind, eine Verarbeitung der gespeicherten Signale auszuführen, um die Menge von Knochengewebe, die auf Höhe der Kontaktgrenzfläche in direktem Kontakt mit der äußeren Oberfläche des Zahnimplantats sind, einzuschätzen, derart, dass die Integration des Zahnimplantats in den Knochen auf Höhe des Knochens kontrolliert wird.

9. Vorrichtung nach Anspruch 8, **dadurch gekennzeichnet, dass** sie ein Anpassungsteil (30) umfasst, das die Form eines Rings aufweist, der an dem Ultraschallwandler (12) fest montiert werden kann, wobei der Ring eine kreisförmige innere Oberfläche (31) aufweist, die dazu bestimmt ist, einerseits an einer Seitenwand des Ultraschallwandlers (12), die sich von der aktiven Oberfläche des Ultraschallwandlers erstreckt, und andererseits an einer Seitenwand des Zahnimplantats (1), die sich von dem freien Ende (1a) des Zahnimplantats erstreckt, anzuliegen.

10. Vorrichtung nach Anspruch 9, **dadurch gekennzeichnet, dass** die kreisförmige innere Oberfläche (31) des Rings eine erste kreisförmige innere Oberfläche (311), die dafür ausgelegt ist, an der Seitenwand des Ultraschallwandlers anzuliegen, aufweist, wobei die erste kreisförmige innere Oberfläche über eine im Wesentlichen ebene Verbindungsoberfläche (312) mit einer zweiten kreisförmigen inneren Oberfläche (313) des Rings verbunden ist, die einen Durchmesser aufweist, der kleiner als jener ist, der durch die erste kreisförmige innere Oberfläche (311) gebildet wird, und die dafür ausgelegt ist, an der Seitenwand des Zahnimplantats (1) anzuliegen, wobei der Ring um den Ultraschallwandler fest montiert ist, derart, dass zwischen der aktiven Oberfläche des Wandlers (12) und der im Wesentlichen ebenen Verbindungsoberfläche (312), die die erste und die zweite kreisförmige innere Oberfläche des Rings verbindet, ein freier Raum (e) bleibt.

11. Vorrichtung nach Anspruch 9 oder 10, **dadurch gekennzeichnet, dass** das Anpassungsteil (30) Klemmmittel (32) umfasst, die dafür ausgelegt sind, die kreisförmige innere Oberfläche (31) des Rings anliegend an der Seitenwand des Ultraschallwandlers (12) zu halten.

12. Vorrichtung nach einem der Ansprüche 8 bis 11, **dadurch gekennzeichnet, dass** die Mittenfrequenz der von dem Ultraschallwandler ausgesendeten Ultraschallwelle wenigstens gleich 8 MHz, vorteilhaft wenigstens gleich 10 MHz, ist.

## Claims

1. A method for controlling the mechanical resistance of a dental implant (1) at least partially inserted into a bone (2), the dental implant having the shape of a substantially cylindrical part made of titanium or of a titanium based alloy and having a free end (1a) emerging from the bone surface at the opposite of an end (1b) of the dental implant embedded into the bone, **characterized in that** it is an ultrasonic echographic method comprising steps wherein :

   - an active surface of an ultrasonic transducer (12) is positioned in contact with said free end (1a) and parallelly to the surface formed by said free end of the dental implant ;
   - by means of said ultrasonic transducer (12), ultrasonic waves propagating into the dental implant (1) are emitted along a propagating direction perpendicular to said active surface of said ultrasonic transducer, from said free end of the dental implant towards its embedded end ;
   - by means of said ultrasonic transducer, reflected ultrasonic waves resulting from the interaction of said ultrasonic waves propagating into the dental implant with a contact interface (4) between the dental implant and the bone, located along a boundary between the dental implant external surface and the bone at the dental implant side walls, are collected as signals ; and
   - said signals are treated to evaluate the quantity of bone tissues in direct contact with the dental implant external surface at said contact interface, in order to control the osseous integration of the dental implant at the bone level.

2. The method according to claim 1, **characterized in that** the central frequency of the emitted ultrasonic wave is selected to be equal to or higher than 8 MHz, advantageously equal to or higher than 10 MHz , and **in that** the band-width, measured at -6 dB, is substantially equal to 80% of the central frequency.

3. The method according to claim 1 or 2, **characterized in that** it is used a piezoelectric ultrasonic transducer (12) having an active surface with dimensions at least equal to those of the surface formed by said dental implant free end (1a).

4. The method according to claim 3, **characterized in that** it is provided an adaptation part (30) that may be assembled interdependently with the ultrasonic transducer (12), having a ring shape comprising a circular internal surface (31) intended, on the one hand, to bear against a ultrasonic transducer (12) side wall extending from said active surface and, on the other hand, against a dental implant (1) side wall extending from the free end (1a) of the dental implant (1), and **in that** said active surface of the ultrasonic transducer is positioned in contact with said dental implant free end by means of said adaptation part (30) assembled interdependently with the ultrasonic transducer (12), so as to make reproducible the positioning of the ultrasonic transducer relatively to the implant.

5. The method according to any of the preceding claims, **characterized in that** the treatment step consists to calculate an average temporal value, on a given time interval, from a plurality of consecutively collected signals, so as to generate an echo signal (ECH) in said given time interval including a plurality of elementary echoes (ECH1...., ECHN) resulting from the interaction of said ultrasonic waves propagating into the implant with said contact interface (4) and to treat said echo signal in order to determine an indicator (I) the value of which is representative of the mechanical properties of said contact interface (4).

6. The method according to claim 5, **characterized in that** the determination of said indicator (I) consists to measure, for each one of said elementary echoes (ECH1..., ECHN) of said echo signal (ECH), the corresponding maximum amplitude of said elementary echo and to average the maximum amplitudes of said elementary echoes, said calculated average value defining the quality of the contact interface mechanical properties in terms of mechanical resistance.

7. The method according to claim 5, **characterized in that** the determination of said indicator (I) consists to measure the corresponding maximum amplitude of the even elementary echoes of said echo signal (ECH), and to average the maximum amplitudes of said even elementary echoes, said calculated average value defining the quality of the contact interface mechanical properties in terms of mechanical resistance.

8. A device for controlling the mechanical resistance of a dental implant (1) at least partially inserted into a bone (2), the dental implant having the shape of a substantially cylindrical part made of titanium or of a titanium based alloy and having a free end (1a) emerging from the bone surface and an end (1b) embedded into the bone, at the opposite of said free end, **characterized in that** it comprises an ultrasonic transducer (12) having an active surface that may be positioned in contact with the dental implant free end, parallelly to a surface defined by said dental implant free end, in order to emit ultrasonic waves propagating inside the dental implant along a propagating direction perpendicular to said ultrasonic transducer active surface, from said free end towards said embedded end of said dental implant, said ultrasonic transducer being able to collect, as signals, reflected ultrasonic waves resulting from the interaction of said ultrasonic waves propagating into the implant with a contact interface (4) between the dental implant and the bone, situated along a boundary between the dental implant external surface and the bone at the side walls of the dental implant, said device further comprising storing means (21) intended to store said collected signals representative of said reflected ultrasonic waves and treatment means (22) intended to carry out a treatment on said stored signals to evaluate the quantity of bone tissues in direct contact with the dental implant external surface at said contact interface, in order to control the osseous integration of the dental implant on the bone level.

9. The device according to claim 8, **characterized in that** it comprises an adaptation part (30) having a ring shape that may be mounted interdependently with the ultrasonic transducer (12), said ring comprising a circular internal surface (31) intended, on the one hand, to bear against an ultrasonic transducer (12) side wall extending from said active surface of the ultrasonic transducer and, on the other hand, against a dental implant (1) side wall extending from the free end (1a) of the dental implant.

10. The device according to claim 9, **characterized in that** the circular internal surface (31) of the ring presents a first circular internal surface (311) that may be applied against said ultrasonic transducer side wall, said first circular internal surface being connected via a substantially flat connection surface (312) to a second circular internal surface (313) of said ring, having a diameter lower than that formed by the first circular internal surface (311), and that may be applied against said side wall of the dental implant (1), the ring being mounted interdependently about the ultrasonic transducer in order to leave a free space (e) between said transducer (12) active surface and said substantially flat connection surface (312) connecting said first and second circular internal surfaces of the ring.

11. The device according to claim 9 or 10, **characterized in that** said adaptation part (30) comprises tightening means (32) able to keep the circular internal surface (31) of the ring applied against said ultrasonic transducer (12) side wall.

12. The device according to any one of claims 8 to 11, **characterized in that** the central frequency of the ultrasonic wave emitted by the ultrasonic transducer is equal to or higher than 8 MHz, advantageously equal to or higher than 10 MHz.

Fig.1

Fig.2

Fig.3

ECH₁   ECH₂....ECHₙ

Signal
ECH

0

10   20   30   40   50   60

ECH₁   ECH₂....ECHₙ

Signal
ECH

0

10   20   30   40   50   60
(temps µs)

Fig.4

Fig.5

Fig.6

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 5392779 A